(19) Europäisches Patentamt European Patent Office Office européen des brevets

(11) **EP 3 686 600 A1**

(12) **EUROPEAN PATENT APPLICATION**
published in accordance with Art. 153(4) EPC

(43) Date of publication:
**29.07.2020 Bulletin 2020/31**

(21) Application number: **18857801.7**

(22) Date of filing: **28.08.2018**

(51) Int Cl.:
**G01N 33/543** (2006.01)          **G01N 33/545** (2006.01)

(86) International application number:
**PCT/JP2018/031822**

(87) International publication number:
**WO 2019/058903 (28.03.2019 Gazette 2019/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.09.2017   JP 2017180169**

(71) Applicant: **Teijin Limited**
**Osaka-shi, Osaka 530-0005 (JP)**

(72) Inventor: **NAMBU, Mami**
**Osaka-shi**
**Osaka 530-0005 (JP)**

(74) Representative: **Cockerton, Bruce Roger**
**Carpmaels & Ransford LLP**
**One Southampton Row**
**London WC1B 5HA (GB)**

(54) **SUBSTRATE FOR CHROMATOGRAPHY MEDIUM, CHROMATOGRAPHY MEDIUM, AND STRIP FOR IMMUNOCHROMATOGRAPH**

(57)     Provided is a substrate for a chromatography medium that is included in an immunochromatographic strip, in which the substrate is formed of a hydrophilic polyolefin microporous membrane; the substrate has a contact angle one second after dropwise addition of water of from 0 degrees to 60 degrees on at least one surface of the substrate; and the substrate has a capillary flow time of from 5 seconds/4 cm to 300 seconds/4 cm.

FIG.1

**Description**

Technical Field

The present invention relates to a substrate for a chromatography medium, a chromatography medium, and an immunochromatographic strip.

Background Art

In recent years, there is an increasing need for point-of-care testing (POCT) as the testing has advantages of, for example, shortening the test time, allowing a doctor to perform immediate judgement and care, and improving communication between a doctor and a patient. An immunochromatographic method is useful for POCT and it allows an easy-and-quick detection of a target substance in a sample by only a simple operation of, for example, directly dropping a sample such as the blood or the urine on a test strip.

In the immunochromatographic method, a substance to be detected in the sample is generally detected by using a specific reaction of an antigen and an antibody against the antigen. An immunochromatographic strip is generally constituted of a sample pad, a conjugate pad, a chromatography medium, and an absorption pad. When a sample is applied to the sample pad, a substance to be detected in the sample specifically binds to a labeled antibody included in the conjugate pad to form a complex. Subsequently, the complex is developed to the downstream of the chromatography medium. The complex is captured by a detection antibody immobilized to the chromatography medium, allowing detection thereof through a color reaction.

Conventionally, as a substrate for chromatography medium, a nitrocellulose membrane is generally used (e.g., see Patent literatures 1 to 5).

Citation List

Patent Literature

Patent Literature 1: Japanese Patent Application Laid-Open (JP-A) No. 2008-292326
Patent Literature 2: Japanese Patent Application Laid-Open (JP-A) No. 2009-150869
Patent Literature 3: Japanese Patent Application Laid-Open (JP-A) No. 2010-261912
Patent Literature 4: Japanese Patent Application Laid-Open (JP-A) No. 2013-174607
Patent Literature 5: Japanese Patent Application Laid-Open (JP-A) No. 2014-62820

SUMMARY OF INVENTION

Technical Problem

However, in the conventional chromatography medium using the nitrocellulose membrane, the nitrocellulose is a material that is prepared by processing a natural fiber. For this reason, the nitrocellulose membrane has large variation in a capillary flow time, thus requiring a complicated operation such as adjusting the capillary flow time by an agent. On the other hand, a test needs to be performed rapidly in the immunochromatographic method.

An embodiment of the present disclosure has been made under the above circumstances.

An object of the disclosure is to provide a substrate for a chromatography medium that is included in an immunochromatographic strip and has small variation in a capillary flow time and an excellent test speed.

Solution to Problem

Specific solution to solve the above issues includes the following aspects.

[1] A substrate for a chromatography medium that is included in an immunochromatographic strip, in which:

the substrate is formed of a hydrophilic polyolefin microporous membrane;
the substrate has a contact angle one second after dropwise addition of water of from 0 degrees to 60 degrees on at least one surface of the substrate; and
the substrate has a capillary flow time of from 5 seconds/4 cm to 300 seconds/4 cm.

[2] The substrate for a chromatography medium according to [1], in which the hydrophilic polyolefin microporous membrane has a membrane thickness of from 20 μm to 450 μm.

[3] The substrate for a chromatography medium according to [1] or [2], in which the hydrophilic polyolefin microporous membrane has a porosity of from 70% to 95%.

[4] The substrate for a chromatography medium according to any one of [1] to [3], in which the hydrophilic polyolefin microporous membrane has a BET specific surface area of from 1 m$^2$/g to 30 m$^2$/g.

[5] The substrate for a chromatography medium according to any one of [1] to [4], in which the hydrophilic polyolefin microporous membrane has a mean flow pore diameter of from 0.02 μm to 5 μm.

[6] The substrate for a chromatography medium according to any one of [1] to [5], in which the hydrophilic polyolefin microporous membrane is a microporous membrane in which a surfactant is attached to at least one of a surface of the polyolefin microporous membrane or an inner surface of a pore of the polyolefin microporous membrane.

[7] The substrate for a chromatography medium according to any one of [1] to [6], in which the hydrophilic polyolefin microporous membrane is a microporous membrane in which a plasma treatment is performed on at least one of a surface of the polyolefin microporous membrane or an inner surface of a pore of the polyolefin microporous membrane.

[8] A chromatography medium including: the substrate for a chromatography medium according to any one of [1] to [7]; and

a detection portion which is arranged at the substrate for a chromatography medium and at which a detection agent that specifically binds to a substance to be detected is immobilized.

[9] An immunochromatographic strip including the chromatography medium according to [8].

Advantageous Effects of Invention

According to the disclosure, there is provided a substrate for a chromatography medium that is included in an immunochromatographic strip and has small variation in a capillary flow time and an excellent test speed.

BRIEF DESCRIPTION OF DRAWINGS

Fig. 1 shows a schematic diagram illustrating an approximate configuration of a general immunochromatographic strip.
Fig. 2 shows a diagram schematically illustrating a cross section of the general immunochromatographic strip.
Fig. 3 shows a diagram schematically illustrating a cross section of the general immunochromatographic strip.
Fig. 4 shows a schematic diagram describing a method of measuring a capillary flow time of a substrate for chromatography medium.

DESCRIPTION OF EMBODIMENTS

An embodiment of the invention will be described below. The description and Examples described below illustrate the embodiment without limiting the scope of the invention. A working mechanism described in the disclosure includes speculation, and validity thereof does not limit the scope of the invention.

In a case where an embodiment of the disclosure is described with reference to a drawing, a configuration of the embodiment is not limited to the one shown in the drawing. Further, a size of member in each drawing is only schematic, and a relative size relationship between members is not limited to the one shown in the drawing.

In the disclosure, the numerical range denoted by using "to" represents the range inclusive of the number written before and after "to" as the minimum and maximum values.

Regarding a numerical range described in a stepwise manner in the disclosure, an upper limit value or a lower limit value described in one numerical range may be replaced with an upper limit value or a lower limit value of another numerical range described in a stepwise manner. Further, regarding a numerical range described in the disclosure, an upper limit value or a lower limit value of the numerical range may be replaced with a value shown in Examples.

The term "step" in the disclosure includes not only an independent step, but also a step that cannot be clearly distinguished from other steps, as long as the intended purpose of the step is achieved.

In the disclosure, in a case where plural kinds of substances that correspond to the same component exist in a composition, the amount of the component in the composition refers to the total amount of the plural kinds of substances existing in the composition unless otherwise specified.

In the disclosure, the "machine direction" means a lengthwise direction of a substrate produced in a long shape, while the "width direction" means a direction orthogonal to the "machine direction". In the disclosure, the "machine direction" is also referred to as an "MD direction", while the "width direction" is also referred to as a "TD direction".

<Substrate for Chromatography Medium>

In the disclosure, there is provided a substrate for a chromatography medium that is included in an immunochromatographic strip (referred to as a "substrate for a chromatography medium" in the disclosure).

The substrate for a chromatography medium in the disclosure is formed of a hydrophilic polyolefin microporous membrane, and the substrate has a contact angle one second after dropwise addition of water of from 0 degrees to 60 degrees on at least one surface of the substrate and that has a capillary flow time of from 5 seconds/4 cm to 300 seconds/4 cm.

According to the disclosure, there is provided the substrate for a chromatography medium having small variation in the capillary flow time and an excellent test speed.

Hereinafter, a constituent element of the substrate for a chromatography medium of the disclosure will be described in detail.

[Hydrophilic Polyolefin Microporous Membrane]

The substrate for a chromatography medium of the disclosure is formed of a hydrophilic polyolefin microporous membrane. The hydrophilic polyolefin microporous membrane is a hydrophilic microporous membrane configured by including a polyolefin. The hydrophilic polyolefin microporous membrane of the disclosure refers to a membrane in which a fibrillar polyolefin forms a three-dimensional network structure having a large number of micropores connected to each other therein, thereby allowing gas or liquid to pass through from one side to the other side of the membrane.

As the polyolefin is a hydrophobic resin, the polyolefin microporous membrane is originally hydrophobic. The hydrophilic polyolefin microporous membrane in the substrate for a chromatography medium of the disclosure is a polyolefin microporous membrane to which hydrophilicity is imparted by a hydrophilic treatment. A hydrophilic treatment method of the polyolefin microporous membrane will be described in detail below.

In the disclosure, hydrophilicity of the polyolefin microporous membrane is defined by having a contact angle one second after dropwise addition of water of 60 degrees or less on at least one surface thereof. The contact angle one second after dropwise addition of water is a value measured by a measuring method described below.

[Contact Angle]

The substrate for a chromatography medium (i.e., the hydrophilic polyolefin microporous membrane) of the disclosure has a contact angle one second after dropwise addition of water of from 0 degree to 60 degrees on at least one surface thereof. When the immunochromatographic strip is configured by the substrate for a chromatography medium of the disclosure, a surface of the substrate for a chromatography medium of the disclosure having a contact angle one second after dropwise addition of water of from 0 degrees to 60 degrees corresponds to the side where a liquid sample possibly containing a substance to be detected is received. Both surfaces of the substrate for a chromatography medium of the disclosure preferably have a contact angle one second after dropwise addition of water of from 0 degrees to 60 degrees.

The contact angle one second after dropwise addition of water on the surface of the substrate for a chromatography medium (i.e., the hydrophilic polyolefin microporous membrane) of disclosure is a value measured by the following measuring method.

The substrate for a chromatography medium is left standing in an atmosphere at a temperature of 24°C and a relative humidity of 60% for 24 hours or more for moisture conditioning, and, then, in the atmosphere at the same temperature and humidity, the static contact angle one second after dropwise addition of water is measured by dropping 1 $\mu$L of water droplet from a syringe onto the surface of the substrate for a chromatography medium using a fully automatic contact angle meter in accordance with the $\theta/2$ method.

The substrate for a chromatography medium (i.e., the hydrophilic polyolefin microporous membrane) of the disclosure has a contact angle one second after dropwise addition of water of 60 degrees or less on at least one surface thereof. This improves a test speed and reduces variation in a capillary flow time. From this point of view, the contact angle one second after dropwise addition of water on the surface of the substrate for a chromatography medium of the disclosure is preferably 50 degrees or less, more preferably 40 degrees or less, still more preferably 30 degrees or less. From the standpoint of improving the test accuracy, the contact angle one second after dropwise addition of water on the surface of the substrate for a chromatography medium of the disclosure is preferably 1 degree or more, more preferably 5 degrees or more.

[Capillary Flow Time]

The substrate for a chromatography medium (i.e., the hydrophilic polyolefin microporous membrane) of the disclosure has a capillary flow time of from 5 seconds/4 cm to 300 seconds/4 cm. The capillary flow time of the substrate for a chromatography medium of the disclosure is a value measured by the following water absorption test.

Water absorption test: the substrate for a chromatography medium is cut out into a rectangular shape having a length of 6 cm in the TD direction and a length of 1 cm in the MD direction. An adhesive tape is adhered to on one end part of the cut-out piece in the lengthwise direction and the cut-out piece is fixed on a polypropylene plate. The plate is tilted such that the lengthwise direction of the cut-out piece forms an angle of 20 degrees relative to the vertical direction and inserted into a beaker containing water with the cut-out piece arranged on the upper side until the other end part of the cut-out piece in the lengthwise direction is immersed in the water in a depth of 1 cm. This state is kept in an atmosphere at normal pressure having a temperature of 24°C and a relative humidity of 60%. Starting from the moment when the cut-out piece is immersed in the water, the time is measured until the water migrates on the cut-out piece by a capillary phenomenon and the entire interface between a part wetted by the water and a part not wetted by the water in the cut-out piece reaches a position apart by 4 cm from the water surface in the beaker in the lengthwise direction of the cut-out piece. This time is defined as the capillary flow time.

The test accuracy of the substrate for a chromatography medium of the disclosure is improved by having a capillary flow time of 5 seconds/4 cm or more. From this point of view, the capillary flow time of the substrate for a chromatography medium is preferably 10 seconds/4 cm or more, more preferably 20 seconds/4 cm or more. The substrate for a chromatography medium of the disclosure having a capillary flow time of 300 seconds/4 cm or less has an excellent test speed and less variation in the capillary flow time. From this point of view, the capillary flow time of the substrate for a chromatography medium is preferably 250 seconds/4 cm or less, more preferably 200 seconds/4 cm or less.

Examples of a method of controlling the contact angle of water and the capillary flow time of the substrate for a chromatography medium (i.e., the hydrophilic polyolefin microporous membrane) of the disclosure include adjustment of the porous structure, the pore diameter, and the porosity of the polyolefin microporous membrane; a method and extent of a hydrophilic treatment of the polyolefin microporous membrane; and a type and adhesion amount of a surfactant adhered to the polyolefin microporous membrane.

Next, characteristics of the hydrophilic polyolefin microporous membrane as the substrate for a chromatography medium of the disclosure and the polyolefin microporous membrane before the hydrophilic treatment will be described. Characteristics that are shared by the hydrophilic polyolefin microporous membrane and the polyolefin microporous membrane before the hydrophilic treatment will be simply described as characteristics of the "polyolefin microporous membrane".

[Mean Flow Pore Diameter]

The mean flow pore diameter of the polyolefin microporous membrane is preferably from 0.02 $\mu$m to 5 $\mu$m. When the polyolefin microporous membrane has a mean flow pore diameter of 0.02 $\mu$m or more, the capillary flow time is within a more preferable range and the test speed can be further improve. From this point of view, the mean flow pore diameter of the polyolefin microporous membrane is more preferably 0.05 $\mu$m or more, still more preferably 0.1 $\mu$m or more. On the other hand, the sufficient test accuracy can be easily obtained when the mean flow pore diameter of the polyolefin microporous membrane is 5 $\mu$m or less. From this point of view, the mean flow pore diameter of the polyolefin microporous membrane is more preferably 4 $\mu$m or less, still more preferably 3 $\mu$m or less.

The mean flow pore diameter of the polyolefin microporous membrane is obtained by a perm porometer (model: CFP-1200-AEXL) manufactured by Porous Materials INC. using a wetting agent, GALWICK (surface tension: 15.9 dynes/cm) manufactured by Porous Materials INC., in accordance with the half-dry method stipulated in ASTM E1294-89.

[Membrane Thickness]

The membrane thickness of the polyolefin microporous membrane is preferably from 20 $\mu$m to 450 $\mu$m. The polyolefin microporous membrane having a membrane thickness of 450 $\mu$m or less is preferable from the standpoint of reducing the sample amount in use or improving the test accuracy. From this point of view, the membrane thickness of the polyolefin microporous membrane is more preferably 300 $\mu$m or less, still more preferably 200 $\mu$m or less. On the other hand, the polyolefin microporous membrane having a membrane thickness of 20 $\mu$m or more is preferable as the capillary flow time is within a more preferable range and the test speed can be improved. From this point of view, the membrane thickness of the polyolefin microporous membrane is more preferably 25 $\mu$m or more, still more preferably 30 $\mu$m or more.

[Porosity]

The porosity of the polyolefin microporous membrane is preferably from 70% to 95%. The polyolefin microporous membrane having a porosity of 70% or more is preferable as the capillary flow time is within a more preferable range and the test speed can be improved. From this point of view, the porosity of the polyolefin microporous membrane is more preferably 75% or more, still more preferably 80% or more. On the other hand, the polyolefin microporous membrane having a porosity of 95% or less is preferable as the mechanical strength of the membrane becomes excellent and handleability thereof is improved. From this point of view, the porosity of the polyolefin microporous membrane is more preferably 93% or less.

The porosity (%) of the polyolefin microporous membrane is obtained by the following Formula.

$$\text{Porosity (\%)} = \{1 - (Wa/xa + Wb/xb + Wc/xc + \cdots + Wn/xn)/t\} \times 100$$

In this Formula, constituent materials of the polyolefin microporous membrane are represented by a, b, c, $\cdots$, n, the mass of the constituent materials are respectively represented by Wa, Wb, Wc, $\cdots$, Wn (g/cm$^2$), the true density of the constituent materials are respectively represented by xa, xb, xc, $\cdots$, xn (g/cm$^3$), and the membrane thickness is represented by t (cm).

[Specific Surface Area]

The BET specific surface area of the polyolefin microporous membrane is preferably from 1 m$^2$/g to 30 m$^2$/g. The polyolefin microporous membrane having a BET specific surface area of 30 m$^2$/g or less is preferable as the capillary flow time is within a more preferable range and the test speed can be improved. From this point of view, the BET specific surface area of the polyolefin microporous membrane is more preferably 25 m$^2$/g or less, still more preferably 20 m$^2$/g or less. On the other hand, the polyolefin microporous membrane having a BET specific surface area of 1 m$^2$/g or more is preferable as the mechanical strength of the membrane becomes excellent and handleability thereof is improved. From this point of view, the BET specific surface area of the polyolefin microporous membrane is more preferably 2 m$^2$/g or more.

The BET specific surface area of the polyolefin microporous membrane is a value obtained by measuring the adsorption isotherm at a relative pressure setting from $1.0 \times 10^{-3}$ to 0.35 by a nitrogen gas adsorption method at the liquid nitrogen temperature using a specific surface area measuring device (model: BELSORP-mini) manufactured by MicrotracBEL Corp., followed by analysis using the BET method.

[Polyolefin]

The polyolefin included in the polyolefin microporous membrane is not particularly limited. However, examples thereof include polyethylene, polypropylene, polybutylene, polymethylpentene, and a copolymer of polypropylene and polyethylene. Of these, polyethylene is preferable, and a high-density polyethylene, a mixture of a high-density polyethylene and an ultra-high molecular weight polyethylene, and the like are preferable. The polyolefin microporous membrane including only polyethylene as a polyolefin is preferable.

The polyolefin microporous membrane is preferably configured from a polyolefin composition (in the disclosure, the polyolefin composition means a composition including two or more kinds of polyolefins, while a composition including only polyethylene as a polyolefin is referred to as a polyethylene composition). The polyolefin composition forms a network structure by fibrillation upon stretching and thus exhibits an effect of increasing the porosity of the polyolefin microporous membrane.

The polyolefin composition preferably includes an ultra-high molecular weight polyethylene having a weight-average molecular weight of $9 \times 10^5$ or more in an amount of preferably from 5% by mass to 30% by mass, more preferably from 5% by mass to 25% by mass, still more preferably from 5% by mass to 15% by mass.

The weight-average molecular weight of the entire polyolefins in the polyolefin composition is preferably from $2 \times 10^5$ to $2 \times 10^6$. The polyolefin composition is preferably prepared by mixing an ultra-high molecular weight polyethylene having a weight-average molecular weight of $9 \times 10^5$ or more and a high-density polyethylene having a weight-average molecular weight of from $2 \times 10^5$ to $8 \times 10^5$ and a density of from 0.92 g/cm$^3$ to 0.96 g/cm$^3$ in a mass ratio of from 5:95 to 30:70.

The weight-average molecular weight of the polyolefin constituting the polyolefin microporous membrane is obtained by dissolving the polyolefin microporous membrane in o-dichlorobenzene under heat and performing a measurement by gel permeation chromatography (system: Alliance GPC 2000 model manufactured by Waters Corp., column:

GMH6-HT and GMH6-HTL) under conditions of a column temperature of 135°C and a flow rate of 1.0 mL/min. Molecular weight monodisperse polystyrene (manufactured by TOSOH Corp.) is used for molecular weight calibration.

[Production Method of Polyolefin Microporous Membrane]

The polyolefin microporous membrane can be produced, for example, by a production method including the following steps (I) to (IV).

Step (I): a step of preparing a solution containing the polyolefin composition including polyethylene and a volatile solvent having a boiling point under atmospheric pressure of less than 210°C.
Step (II): a step of obtaining a first gel-like molded product by melting and kneading the solution, extruding the resulting melt-kneaded product through a die, and solidifying the extrudate by cooling.
Step (III): a step of obtaining a second gel-like molded product by stretching (primary stretching) the first gel-like molded product in at least one direction and drying the solvent.
Step (IV): a step of stretching (secondary stretching) the second gel-like molded product in at least one direction.

The step (I) is a step of preparing a solution containing the polyolefin composition and a volatile solvent having a boiling point under atmospheric pressure of less than 210°C. The solution is preferably a thermo-reversible sol-gel solution, which is prepared by dissolving the polyolefin composition in the solvent under heat and thereby forming a sol. The volatile solvent having a boiling point under atmospheric pressure of less than 210°C is not particularly limited as long as the solvent can sufficiently dissolve the polyolefin. Examples of the volatile solvent include tetralin (206°C to 208°C), ethylene glycol (197.3°C), decalin (decahydronaphthalene, 187°C to 196°C), toluene (110.6°C), xylene (138°C to 144°C), diethyl triamine (107°C), ethylenediamine (116°C), dimethyl sulfoxide (189°C), and hexane (69°C) (the temperature in parentheses represents a boiling point under atmospheric pressure). Of these, decalin or xylene is preferable. The volatile solvent may be used singly, or in combination of two or more kinds thereof.
In the solution prepared in the step (I), the concentration of the polyolefin composition is preferably from 10% by mass to 40% by mass, more preferably from 15% by mass to 35% by mass, from the standpoint of controlling the capillary flow time of the polyolefin microporous membrane. Having the concentration of the polyolefin composition of 10% by mass or more can prevent the occurrence of cutting in the membrane forming step of the polyolefin microporous membrane and enhance the mechanical strength of the polyolefin microporous membrane, resulting in improvement of handleability thereof. Having the concentration of the polyolefin composition of 40% by mass or less facilitates the formation of the pores of the polyolefin microporous membrane.
The step (II) is a step of obtaining the first gel-like molded product by melting and kneading the solution prepared in the step (I), extruding the resulting melt-kneaded product through a die, and solidifying the extrudate by cooling. In the step (II), for example, the extrudate is obtained by performing extrusion from a die in a temperature range from the melting point of the polyolefin composition to the melting point + 65°C, and then the extrudate is cooled down to obtain the first gel-like molded product. The first gel-like molded product is preferably formed into a sheet shape. Cooling may be performed by immersion in water or an organic solvent or through contact with a cooled metal roll. In general, cooling is performed by immersion in the volatile solvent used in the step (I).
The step (III) is a step of obtaining the second gel-like molded product by stretching (primary stretching) the first gel-like molded product in at least one direction and drying the solvent. The stretching step in the step (III) is preferably performed by biaxial stretching. As the biaxial stretching, successive biaxial stretching in which longitudinal stretching and transverse stretching are separately conducted or simultaneous biaxial stretching in which longitudinal stretching and transverse stretching are simultaneously conducted may be used. A stretching ratio (the product of the longitudinal stretching ratio and the transverse stretching ratio) of the primary stretching is preferably from 1.1 times to 3 times from the standpoint of controlling the capillary flow time of the polyolefin microporous membrane, and a temperature at the time of stretching is preferably 75°C or less. The drying step in the step (III) is performed at any temperature without particular limitation as long as the second gel-like molded product is not deformed. However, the drying step is preferably performed at 60°C or less.
The stretching step and the drying step in the step (III) may be performed simultaneously or step by step. For example, the primary stretching may be performed while pre-drying is performed, followed by main drying, or the primary stretching may be performed between pre-drying and main drying. The primary stretching can also be performed in a state where the solvent remains at an appropriately level by controlling drying.
The step (IV) is a step of stretching (secondary stretching) the second gel-like molded product in at least one direction. The stretching step in the step (IV) is preferably performed by biaxial stretching. The stretching step in the step (IV) may be performed by any of: successive biaxial stretching in which longitudinal stretching and transverse stretching are separately conducted; simultaneous biaxial stretching in which longitudinal stretching and transverse stretching are simultaneously conducted; a step of conducting stretching several times in the longitudinal direction and then conducting stretching in the transverse direction; a step of conducting stretching in the longitudinal direction and then conducting stretching several times in the transverse direction; or a step of conducting successive biaxial stretching and then further conducting stretching once or several times in the longitudinal direction and/or in the transverse direction.

The stretching ratio (the product of the longitudinal stretching ratio and the transverse stretching ratio) of the secondary stretching is preferably from 5 times to 65 times, more preferably from 8 times to 55 times, from the standpoint of controlling the capillary flow time of the polyolefin microporous membrane. The larger stretching ratio tends to increase the occurrence frequency of cutting in the membrane formation of the polyolefin microporous membrane. The smaller stretching ratio tends to increase thickness irregularity. The stretching is performed after the solvent is removed. However, the stretching can also be performed in a state where the solvent remains at an appropriately level by controlling drying. The stretching temperature of the secondary stretching is preferably from 90°C to 135°C, more preferably from 90°C to 125°C, from the standpoint of controlling the capillary flow time of the polyolefin microporous membrane.

A heat fixing treatment may be performed after the step (IV). The heat fixing temperature is preferably from 120°C to 160°C, more preferably from 125°C to 150°C, from the standpoint of controlling the capillary flow time of the polyolefin microporous membrane. The higher heat fixing temperature increases the occurrence frequency of cutting in the membrane formation of the polyolefin microporous membrane.

The above production method makes it possible to produce the polyolefin microporous membrane having a highly porous structure.

[Hydrophilic Treatment of Polyolefin Microporous Membrane]

Examples of the hydrophilic treatment method of the polyolefin microporous membrane include a corona discharge treatment, a plasma treatment, a UV/ozone treatment, coating of a surfactant or a hydrophilic material, and graft polymerization of a hydrophilic monomer.

The plasma treatment is preferably performed on a surface of the polyolefin microporous membrane and/or an inner surface of a pore of the polyolefin microporous membrane from the standpoint of improving the detection accuracy. The polyolefin microporous membrane is preferably made hydrophilic by a treatment with a surfactant from the standpoint of reducing variation in the capillary flow time of the polyolefin microporous membrane. That is, a surfactant is preferably attached to a surface of the polyolefin microporous membrane and/or an inner surface of a pore of the polyolefin microporous membrane from the standpoint of reducing variation in the capillary flow time of the polyolefin microporous membrane.

In one embodiment of the hydrophilic polyolefin microporous membrane, a plasma treatment is performed on at least one of a surface of the polyolefin membrane or an inner surface of a pore of the polyolefin microporous membrane and a surfactant is attached to at least one of a surface of the polyolefin membrane or an inner surface of a pore of the polyolefin microporous membrane. The present embodiment is preferred from the standpoint of achieving both the improvement of the detection accuracy and the reduction in variation in the capillary flow time.

As the surfactant used for the surface treatment of the polyolefin microporous membrane, any of a cationic surfactant, an anionic surfactant, an ampholytic surfactant, or a nonionic surfactant can be used.

Examples of the cationic surfactant include a higher amine halogenate, an alkylpyridinium halide, and a quaternary ammonium salt.

Examples of the anionic surfactant include a higher fatty acid alkaline salt, a polyoxyethylene alkyl ether sulfonic acid ester salt, a polyoxyethylene alkyl ether phosphonic acid salt, an alkyl sulfate, an alkylbenzene sulfate, an alkyl sulfonate, an alkylaryl sulfonate, and a sulfosuccinic acid ester salt. Of these, an alkylbenzene sulfate is preferable and sodium dodecylbenzenesulfonate (SDBS) is particularly preferable.

Examples of the ampholytic surfactant include an alkylbetaine compound, an imidazoline compound, an alkylamine oxide, and a bisoxyborate compound.

Examples of the nonionic surfactant include a polyoxyethylenealkyl ether, a polyoxyethylenealkylphenyl ether, a polyoxyethylenealkylallyl ether, a glycerin fatty acid ester, a polyoxyethylene sorbitan fatty acid ester, and a sorbitan fatty acid ester.

Examples of the hydrophilic material for coating the polyolefin microporous membrane include cellulose, a polyvinyl alcohol, a polyethylene-polyvinyl alcohol copolymer, polyurethane, and polyacrylamide.

Examples of the hydrophilic monomer that is graft-polymerized on the surface of the polyolefin microporous membrane include acrylic acid, methacrylic acid, a vinyl alcohol, N-vinyl-2-pyrrolidone, and vinylsulfonic acid.

<Immunochromatographic Strip>

The immunochromatographic strip of the disclosure is for testing a liquid sample possibly containing a substance to be detected.

In a preferred embodiment, the immunochromatographic strip of the disclosure includes

a sample pad for receiving the sample,

a conjugate pad including a labeling substance that specifically binds to the substance to be detected, and

a chromatography medium at which a detection agent that specifically binds to the substance to be detected is immobilized.

The shape and width of the immunochromatographic strip of the disclosure is not particularly limited as long as the strip has an easy-to-operate shape and width.

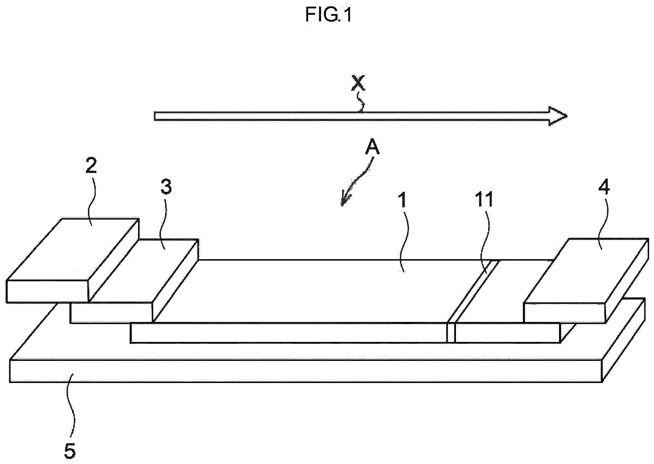

Fig. 1 shows a configuration example of the immunochromatographic strip of the disclosure. An immunochromatographic strip A has a configuration in which a sample pad 2 for receiving a sample dropped thereon, a conjugate pad 3 including a labeling substance, a chromatography medium 1 at which a detection agent that specifically binds to a substance to be detected is immobilized, and an absorption pad 4 for absorbing the excessive sample are fixed to a long support plate 5 made of a plastic or the like in this order from the upstream to the downstream of the developing direction (a direction indicated by an arrow X in Fig. 1).

The chromatography medium 1 includes the substrate for a chromatography medium of the disclosure and a detection portion 11 arranged at the substrate for a chromatography medium. The detection portion 11 is a region where the detection agent that specifically binds to the substance to be detected is immobilized. The substrate for a chromatography medium is preferably lined with a film by backing processing before being used in the chromatography medium 1.

The chromatography medium 1 includes the substrate for a chromatography medium of the disclosure. In an example of the chromatography medium 1, the TD direction of the substrate for a chromatography medium of the disclosure corresponds to the developing direction of the sample (the direction indicated by an arrow X in Fig. 1). In another example of the chromatography medium 1, the MD direction of the substrate for a chromatography medium of the disclosure corresponds to the developing direction of the sample (the direction indicated by an arrow X in Fig. 1).

As shown in Fig. 1, the detection portion 11 is preferably formed in a linear shape in the direction orthogonal to the developing direction at a desired position in the substrate for a chromatography medium. However, the shape of the detection portion 11 is not limited to a linear shape, and the detection portion 11 may be formed in a shape of, for example, a circular spot, a numeral, a character, or a symbol (e.g., + and -).

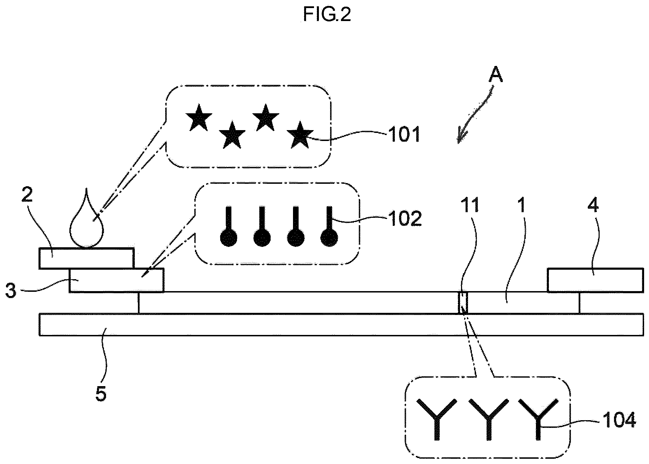

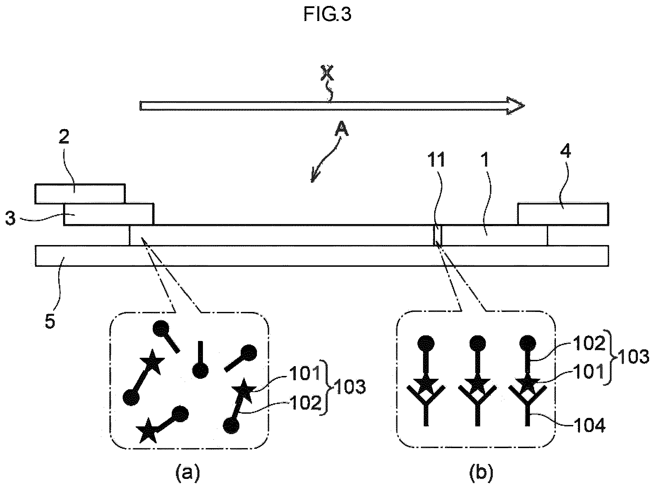

A method of using the immunochromatographic strip A will be described with reference to Fig. 2 and Fig. 3. Fig. 2 and Fig. 3 each show a diagram schematically illustrating a cross section of the immunochromatographic strip A in the thickness direction. When the sample possibly containing a substance to be detected 101 is dropped on the sample pad 2, the sample migrates to the conjugate pad 3 while an unnecessary component for the measurement is removed, if appropriate. The conjugate pad 3 includes a labeling substance 102 that contains a binding portion (e.g., an antibody) capable of binding to the substance to be detected 101 in the sample. In the conjugate pad 3, the labeling substance 102 binds to the substance to be detected 101 to form a complex 103. The samples including the complex 103 migrate from the conjugate pad 3 to the chromatography medium 1 (a state shown in Fig. 3(a)). The samples in the chromatography medium 1 migrate toward the detection portion 11 including the detection agent 104. If the sample contains the substance to be detected 101, the complex 103 specifically binds to the detection agent 104 in the detection portion 11, causing concentration of the labeling substances 102 in the detection portion 11 (a state shown in Fig. 3(b)). The concentrated labeling substances 102 are detected by visual observation or using an apparatus, thereby making it possible to qualitatively and quantitatively analyze the presence of the substance to be detected 101 in the sample. Subsequently, the excessive sample is absorbed by the absorption pad 4.

In the immunochromatography using the immunochromatographic strip of the disclosure, a developing liquid may be used as needed. The developing liquid is a liquid constituting a mobile phase in the immunochromatography method and migrates along with the sample containing the substance to be detected through the chromatography medium. Any liquid having such properties may be used as the developing liquid. The developing liquid may be applied to the chromatography medium via the same path as that of the sample or via a different path from that of the sample.

Examples of an application of the immunochromatographic strip include diagnosis of an infectious disease such as influenza, hepatitis B, or food poisoning; a pregnancy test; and diagnosis using biomarkers for various diseases including heart attack.

Hereinafter, a sample which can be analyzed by the immunochromatography and a member included in the immunochromatographic strip will be described in detail.

[Sample]

The sample which can be analyzed by the immunochromatographic strip of the disclosure is not limited as long as the sample possibly contains a substance to be detected. Examples of the sample include a biological sample such as a body fluid of an animal (particularly human) (e.g., blood, serum, plasma, spinal fluid, lacrimal fluid, sweat, urine, pus, nasal mucus, or sputum); an excrement (e.g., feces) of an animal (particularly human); an organ, a tissue, a mucosa, or skin of an animal (particularly human), or a swab specimen, a swab, or a gargle sample which may contain such a substance; an animal or plant itself; a dried animal or plant; a liquid plant extract; or a liquid food extract.

The substance to be detected is not particularly limited as long as there is a substance that specifically binds to the substance to be detected. Examples of the substance to be detected include a protein, a peptide, a nucleic acid, a saccharide, a glycoprotein, a glycolipid, and a glycoconjugate. In the disclosure, the term "specifically binding" means that binding is based on affinity of a biomolecule. Examples of the binding based on such affinity include a binding between an antigen and an antibody, a binding between of a saccharide and a lectin, a binding between of a hormone and a receptor, a binding between of an enzyme and an inhibitor, a binding between complementary nucleic acids, and a binding between a nucleic acid and a nucleic acid binding protein. In a case where the substance to be detected has antigenicity, examples of the substance that specifically binds to the substance to be detected include a polyclonal antibody and a monoclonal antibody. In a case where the substance to be detected is a saccharide or a glycoprotein, examples of the substance that specifically binds to the substance to be detected include a lectin. Specific examples of the substance to be detected include, without being limited thereto, carcinoembryonic antigen (CEA), HER2 protein, prostate-specific antigen (PSA), CA19-9, $\alpha$-fetoprotein (AFP), immunosuppressive acidic protein (IAP), CA15-3, CA125, troponin I, troponin T, CK-MB, C reactive protein (CRP), an estrogen receptor, a progesterone receptor, human chorionic gonadotropin (hCG), luteinizing hormone (LH), follicle stimulating hormone (FSH), human hemoglobin, albumin, glycated albumin, fecal occult blood, a syphilis antibody, a chlamydiae antigen, a group A $\beta$-streptococcal antigen, a HBs antibody, a HBs antigen, an influenza virus, a rotavirus, and an adenovirus.

The sample dropped on the sample pad may be any of the following (i) to (v).

(i) a biological sample itself.
(ii) a liquid extract extracted from a biological sample using an extraction solvent.
(iii) a diluted liquid prepared by diluting a biological sample or a liquid extract with a diluent.
(iv) a concentrated liquid prepared by concentrating a biological sample or a liquid extract.
(v) a liquid including a complex prepared by mixing any of (i) to (iv) with the labeling substance and allowing the labeling substance to bind to a substance to be detected.

As the extraction solvent or the diluent, a solvent used in a common immunological analysis method (e.g., water, physiological saline, a buffer solution, etc.) can be used.

[Sample Pad]

Example of the sample pad include nonwoven fabric, woven fabric, a paper-like sheet, and a microporous membrane, made of one or two or more kinds of various fibers such as cellulose, glass, polyurethane, polyacetate, cellulose acetate, nylon, polyolefin, and cotton. The sample pad not only receives the sample, but also has a function of filtering an insoluble particle or the like in the sample. In order to prevent a reduction in the test accuracy caused when the substance to be detected in the sample is nonspecifically absorbed to the sample pad, the sample pad may be subjected to a non-specific adsorption-preventing treatment in advance. The sample pad may be formed not only as a single sheet made of the above nonwoven fabric or the like, but also as a layered body in which the above nonwoven fabric or the like and a blood cell separation membrane or the like are placed one over the other.

[Conjugate Pad]

The conjugate pad is preferably a porous membrane including the labeling substance that specifically binds to the substance to be detected in the sample. The conjugate pad can be produced by immersing the porous membrane in a suspension including the labeling substance and drying the porous membrane. Examples of the porous membrane constituting the conjugate pad include nonwoven fabric, woven fabric, a paper-like sheet, and a microporous membrane, made of one or two or more kinds of various fibers such as cellulose, glass, polyurethane, polyacetate, cellulose acetate, nylon, polyolefin, and cotton.

[Labeling Substance]

The labeling substance includes a binding portion that specifically binds to the substance to be detected and a labeling portion that is detected by visual observation or using an apparatus.

The binding portion of the labeling substance may be selected in accordance with the substance to be detected as a target, if appropriate. Examples of a combination of the substance to be detected and the binding portion of the labeling substance (substance to be detected/binding portion of labeling substance) include antigen/antibody, saccharide/lectin, glycoprotein/lectin, hormone/hormone receptor, enzyme/enzyme inhibitor, nucleic acid/complementary nucleic acid, and nucleic acid/nucleic acid binding protein.

As the labeling portion of the labeling substance, an insoluble carrier, an enzyme, or the like can be mentioned.

Of these, an insoluble carrier is easily detected by visual observation and is thus preferable. In a case where an insoluble carrier is used as the labeling portion of the labeling substance, the labeling substance can be prepared by sensitizing the insoluble carriers with the binding portion.

Examples of the insoluble carrier include a colloidal metal particle such as gold, silver, or platinum, a colloidal metal oxide particle such as iron oxide, a colloidal nonmetal particle such as sulfur, and a latex particle formed by a synthetic polymer. The insoluble carrier is preferably gold colloid from the standpoint of easy detection.

The insoluble carrier is preferably colored from the standpoint of easy detection by visual observation. A colloidal metal particle and a colloidal metal oxide particle each exhibits a specific color by itself in accordance with the particle diameter, thus the color can be used for labeling.

Examples of the colloidal metal particle or the colloidal metal oxide particle include a colloidal gold particle, a colloidal silver particle, a colloidal platinum particle, a colloidal iron oxide particle, and a colloidal aluminum oxide particle. The mean particle diameter of the colloidal metal particle or the colloidal metal oxide particle is preferably from 1 nm to 500 nm, more preferably from 10 nm to 150 nm, still more preferably from 20 nm to 100 nm, from the standpoint of obtaining a strong color tone. The colloidal gold particle and the colloidal silver particle exhibit a red color and a yellow color, respectively, at their appropriate particle diameters and are thus preferable. The colloidal gold particle may be a commercially available particle or prepared by a common method (e.g., a method of reducing chloroauric acid with sodium citrate).

Examples of a method of sensitizing the colloidal metal particle or the colloidal metal oxide particle with the binding portion include a known method that uses physical adsorption or a chemical bond. For example, the labeling substance in which the colloidal gold particle is sensitized with an antibody is prepared by adding antibodies to a dispersion in which gold particles are dispersed in a colloidal form to cause physical adsorption of the antibodies and then blocking the particle surfaces to which the antibodies are not bound by adding a bovine serum albumin solution.

[Chromatography Medium]

The chromatography medium includes the substrate for a chromatography medium and the detection portion which is arranged at the substrate for a chromatography medium and at which a detection agent that specifically binds to the substance to be detected is immobilized. The detection portion is a region where a detection agent that specifically binds to the substance to be detected is immobilized. In a case where there are multiple kinds of the substances to be detected, a plurality of the detection portions at which the detection agents corresponding to each of the substances to be detected are immobilized may be formed in the substrate for a chromatography medium.

The detection agent is a substance that specifically binds to the substance to be detected. Examples of a combination of the substance to be detected, the binding portion of the labeling substance, and the detection agent (substance to be detected/binding portion of labeling substance/detection agent) include antigen/first antibody against antigen/second antibody against antigen, saccharide/first lectin/second lectin, glycoprotein/antibody against glycoprotein/lectin, glycoprotein/lectin/antibody against glycoprotein, hormone/hormone receptor/antibody against hormone, hormone/antibody against hormone/hormone receptor, enzyme/enzyme inhibitor/antibody against enzyme, enzyme/antibody against enzyme/enzyme inhibitor, nucleic acid/first complementary nucleic acid/second complementary nucleic acid, nucleic acid/complementary nucleic acid/nucleic acid binding protein, and nucleic acid/nucleic acid binding protein/complementary nucleic acid.

As a method for immobilizing the detection agent at the substrate for a chromatography medium, either a method in which the detection agent is directly immobilized to the substrate for a chromatography medium physically or chemically, or a method in which the detection agent is bound to a microparticle such as a latex particle physically or chemically and this microparticle is immobilized at the substrate for a chromatography medium (i.e., an indirect immobilization method) may be used. The direct immobilization is preferable from the standpoint of easy adjustment of sensitivity.

Examples of the method in which the detection agent is directly immobilized at the substrate for a chromatography medium include physical adsorption and a covalent bond. In a case where the detection agent is immobilized at the substrate for a chromatography medium by a covalent bond, cyanogen bromide, glutaraldehyde, carbodiimide, or the like can be used as a binding agent.

Examples of the method in which the detection agent is indirectly immobilized at the substrate for a chromatography medium include a method in which an insoluble particle at which the detection agent is bound is immobilized at the substrate for a chromatography medium. As the insoluble particle, a microparticle having a particle diameter (e.g., a mean particle diameter of about 5 $\mu$m or more) that causes the microparticle to be captured by the substrate for a chromatography medium and thereby prevents the movement of the microparticle is selected. As the insoluble particle, various particles used in an antigen-antibody reaction are known. Examples thereof include a microparticle of an organic polymer such as polystyrene, a styrene-butadiene copolymer, a styrene-methacrylic acid copolymer, poly (glycidyl methacrylate), or an acrolein-ethylene glycol dimethacrylate copolymer; a microparticle of gelatin, bentonite, agarose, cross-linked dextran, or the like; a particle of an inorganic oxide such as silica, silica-alumina, or alumina; and an inorganic particle in which a functional group is introduced to an inorganic oxide particle by a silane coupling agent or the like.

Examples of a means of applying the detection agent or the insoluble particle at which the detection agent was bound to the substrate for a chromatography medium include a microsyringe, a pen with an adjustment pump, and ink jet print.

After the detection agent is immobilized to the substrate for a chromatography medium, the substrate for a chromatography medium may be subjected to a blocking treatment by a known method in order to prevent a reduction in accuracy of analysis due to nonspecific absorption. In general, a protein such as bovine serum albumin, skim milk, casein, or gelatin is preferably used for the blocking treatment. After the blocking treatment, the substrate for a chromatography medium may be washed with a surfactant such as Tween 20, Triton X-100, SDS (sodium lauryl sulfate), or SDBS (sodium dodecylbenzene sulfonate), if necessary.

The chromatography medium may further include a control portion, which is a region where a control substance that specifically binds to the labeling substance is immobilized, in the downstream of the detection portion. In a case where the chromatography medium includes the control portion in the downstream of the detection portion, when the sample migrates to the control portion after passing through the detection portion, the labeling substance not captured by the detection portion (i.e., the labeling substance not bound to the substance to be detected) specifically binds to the control substance, resulting in concentration of the labeling substances in the control portion. This allows a user to confirm that the sample migrates to the control portion by visual observation or using an appropriate apparatus and grasp the completion of the test. Examples of the control substance include an antibody against the binding portion of the labeling substance.

[Absorption Pad]

The absorption pad is a member that absorbs the sample which has flown to the downstream in the developing direction. As the absorption pad, for example, an absorbing material such as a filter paper, nonwoven fabric, fabric, or a cellulose acetate membrane is used. The developing speed after the front edge of the sample migrating through the chromatography medium reaches the absorption pad varies depending on the material and the size of the absorption pad, thus the developing speed appropriate for detecting the substance to be detected can be set by selecting the material and the size of the absorption pad.

[Examples]

The substrate for a chromatography medium of the disclosure will be described further in detail below by way of Examples. It should be noted that the material, the use amount, the proportion, the processing procedure and the like in the following Examples can be optionally modified unless the gist of the disclosure is not deviated. Accordingly, the scope of the substrate for a chromatography medium of the disclosure should not be limitedly construed by the following specific examples.

<Method of Measuring Physical Property of Substrate for a chromatography Medium>

Measuring methods applied to the substrate for a chromatography medium (hereinafter, also referred to as "substrate") are as follows.

[Membrane Thickness]

The membrane thickness of the substrate was obtained as an average of thickness values measured at 20 points by a contact-type membrane thickness meter (manufactured by Mitutoyo Corp.). As a contact terminal, a cylindrical terminal having a bottom surface with a diameter of 0.5 cm was used. The measurement pressure was set to 0.1 N.

[Basis Weight]

The basis weight (mass per square meter, $g/m^2$) of the substrate was obtained by measuring the mass of the substrate cut out into a 10 cm $\times$ 10 cm square and dividing the mass by the area.

[Porosity]

The porosity $\varepsilon$ (%) of the substrate was obtained by the following Formula.

$$\epsilon = \{1 - (Wa/xa + Wb/xb + Wc/xc + \cdots + Wn/xn)/t\} \times 100$$

In the Formula, the constituent materials are represented by a, b, c, $\cdots$, n, the mass of the constituent materials are respectively represented by Wa, Wb, Wc, $\cdots$, Wn (g/cm$^2$), the true density of the constituent materials are respectively represented by xa, xb, xc, $\cdots$, xn (g/cm$^3$), and the membrane thickness is represented by t (cm).

[Gurley Value]

The air permeation time T (seconds/100 mL) of the substrate having an area of 642 mm$^2$ was measured in accordance with JIS P 8117 (2009) and the air permeation time $\tau$ (seconds/100 mL/$\mu$m) per 1-$\mu$m thickness was obtained by the following Formula.

$$\tau = T/t$$

T: air permeation time (seconds/100 mL) measured in accordance with JIS P 8117: 2009, t: membrane thickness of substrate ($\mu$m).

[BET Specific Surface Area]

As a pretreatment of the substrate, the substrate was subjected to vacuum deaeration at room temperature using a pretreatment instrument for adsorption measurement (Belprep vac-II) manufactured by MicrotracBEL Corp. before the measurement. The adsorption isotherm at a relative pressure setting from $1.0 \times 10^{-3}$ to 0.35 was measured by a nitrogen gas adsorption method at a liquid nitrogen temperature using a specific surface area measuring apparatus (model: BELSORP-mini) manufactured MicrotracBEL Corp. as a measuring apparatus. The BET specific surface area (m$^2$/g) of the substrate was obtained through the analysis using the BET method.

[Mean Flow Pore Diameter]

The mean flow pore diameter ($\mu$m) of the substrate was obtained by a perm porometer (model: CFP-1200-AEXL) manufactured by Porous Materials INC. using a wetting agent, GALWICK (surface tension: 15.9 dynes/cm) manufactured by Porous Materials INC., in accordance with the half-dry method stipulated in ASTM E1294-89. The measurement temperature was 25°C and the measurement pressure was changed in a range of from 0 to 150 psi.

[Contact Angle]

The contact angle one second after dropwise addition of water on the surface of the substrate was measured using a fully automatic contact angle meter DMs-401 manufactured by Kyowa Interface Science Co., Ltd. and an analyzing software FAMAS (interFAce Measurement & Analysis System). In an atmosphere at normal atmospheric pressure having a temperature of 24°C and a relative humidity of 60%, 1 $\mu$L of water (ion exchange water) was dropped on the substrate and a static contact angle one second after dropwise addition was measured. A syringe with a 22G needle made of SUS (stainless steel) was used for forming a water droplet. The contact angle of water was measured on the surface to which a plasma treatment was performed for the substrates of Example 8 and Example 9.

[Capillary Flow Time]

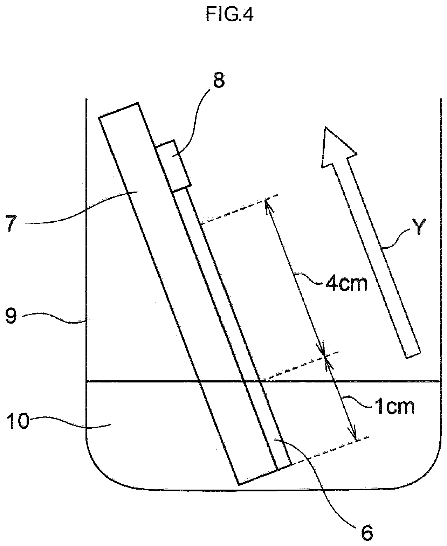

The capillary flow time of the substrate was measure by the following water absorption test. Fig. 4 shows a schematic diagram of the water absorption test.

The substrate was cut into a rectangle having a length of 6 cm in the TD direction and a length of 1 cm in the MD direction to obtain a cut-out piece (referred to as a cut-out piece 6). An adhesive tape 8 was adhered to one end part of the cut-out piece 6 in the lengthwise direction and the cut-out piece 6 was fixed on a polypropylene plate 7 (a length of 121 mm and a width of 52 mm). The plate 7 was tilted such that the lengthwise direction of the cut-out piece 6 formed an angle of 20 degrees relative to the vertical direction and inserted into a beaker 9 containing water 10 (ion exchange water) with the cut-out piece 6 arranged on the upper side until the other end part of the cut-out piece 6 in the lengthwise direction was immersed in the water 10 in a depth of 1 cm. This state was kept under normal atmospheric pressure at a temperature of 24°C and a relative humidity of 60%. Starting from the moment when the cut-out piece 6 was immersed in the water 10, the time was measured until the water migrated on the cut-out piece 6 in a direction indicated by an arrow Y by a capillary phenomenon and the entire interface between a part wetted by the water and a part not wetted by the water in the cut-out piece 6 reaches a position apart by 4 cm from the water surface of the water 10 in the lengthwise direction of the cut-out piece 6. This time was defined as the capillary flow time. An average value of the capillary flow time was obtained by preparing five cut-out pieces 6 and performing the water absorption test using each of the cut-out pieces 6.

[Variance of Capillary Flow Time]

The variance of the capillary flow time was obtained from the capillary flow time of each of the five cut-out pieces 6 by the following Formula.

$$ s = \sqrt{\frac{1}{n} \sum (x_i - \bar{x})^2} $$

s: variance of capillary flow time, n: number of measurements of capillary flow time; $x_i$: each measurement value of capillary flow time, x bar: average value of capillary flow time

<Production of Substrate for a chromatography Medium>

[Example 1]

A polyethylene composition was prepared by mixing 1.25 parts by mass of ultra-high molecular weight polyethylene (hereinafter referred to as "UHMWPE") having a weight-average molecular weight of 4.6 million and 23.75 parts by mass of high-density polyethylene (hereinafter referred to as "HDPE") having a weight-average molecular weight of 0.56 million and a density of 950 kg/m$^3$. A polyethylene solution was prepared by mixing the polyethylene composition and decalin so as to have a polymer concentration of 25% by mass. The polyethylene solution was extruded into a sheet shape from a die at a temperature of 148°C and then the extrudate was cooled in a water bath having a water temperature of 20°C to obtain a first gel-like sheet.

The first gel-like sheet was pre-dried in an atmosphere at a temperature of 70°C for 10 minutes and then subjected to primary stretching at 1.4 times in the MD direction, followed by main drying in an atmosphere at a temperature of 57°C for 5 minutes, to obtain a second gel-like sheet (base tape) (a residual amount of the solvent in the second gel-like sheet was kept less than 1%). Next, as secondary stretching, the second gel-like sheet (base tape) was stretched in the MD direction with a stretch ratio of 3.0 times at a temperature of 100°C and then stretched in the TD direction with a stretch ratio of 9.0 times at a temperature of 125°C, immediately followed by the heat treatment (heat fixation) at 127°C, to obtain a biaxially stretched polyethylene microporous membrane.

A plasma treatment (AP-300 manufactured by Nordson MARCH: output of 150 W, treatment pressure of 400 mTorr, gas flow of 160 sccm, treatment time of 135 seconds) was performed to both surfaces of the polyethylene microporous membrane described above.

The polyethylene microporous membrane subjected to the plasma treatment was immersed in an aqueous solution of 1% by mass sodium dodecylbenzenesulfonate (SDBS) and dried at normal temperature for 24 hours to perform SDBS coating of the polyethylene microporous membrane.

After the plasma treatment and the SDBS coating described above, a hydrophilic polyethylene microporous membrane, that is, the substrate for a chromatography medium was obtained.

A PET film to which an adhesive was attached was stuck on one surface of the substrate for a chromatography medium described above to obtain a layered body.

[Example 2]

The substrate for a chromatography medium and the layered body were produced in the same manner as in Example 1 except that the SDBS coating was not performed to the polyethylene microporous membrane subjected to the plasma treatment.

[Example 3]

The substrate for a chromatography medium and the layered body were produced in the same manner as in Example 1 except that the stretching ratio in the MD direction in the primary stretching was changed to 1.8 times, and, in the secondary stretching, the stretching ratio in the MD direction was changed to 2.0 times, the stretching temperature in the TD direction was changed to 120°C, and the stretching ratio in the TD direction was changed to 4.4 times.

[Example 4]

The substrate for a chromatography medium and the layered body were produced in the same manner as in Example 1 except that the stretching ratio in the MD direction in the primary stretching was changed to 1.2 times, the stretching temperature in the MD direction in the secondary stretching was changed to 90°C, and the temperature of the heat treatment (heat fixation) was changed to 145°C.

[Example 5]

The substrate for a chromatography medium and the layered body were produced in the same manner as in Example 4 except that the SDBS coating was not performed to the polyethylene microporous membrane subjected to the plasma treatment.

[Example 6]

The substrate for a chromatography medium and the layered body were produced in the same manner as in Example 1 except that the polyethylene composition prepared by mixing 3.75 parts by mass of UHMWPE and 21.25 parts by mass of HDPE was used, the die temperature was changed to 152°C, the stretching ratio in the MD direction in the primary stretching was changed to 1.2 times, in the secondary stretching, the stretching temperature in the MD direction was changed to 90°C and the stretching ratio in the MD direction was changed to 4.0 times, and the temperature of the heat treatment (heat fixation) was changed to 144°C.

[Example 7]

The polyethylene composition was prepared by mixing 4.05 parts by mass of UHMWPE and 22.95 parts by mass of HDPE. The polyethylene solution was prepared by mixing the polyethylene composition and decalin so as to have a polymer concentration of 27% by mass. The substrate for a chromatography medium and the layered body were produced in the same manner as in Example 1 except that the polyethylene solution described above was used, the stretching temperature in the MD direction in the secondary stretching was changed to 90°C, and the temperature of the heat fixation was changed to 144°C.

[Example 8]

The substrate for a chromatography medium was produced in the same manner as in Example 1 except that the die temperature was changed to 152°C, the stretching temperature in the MD direction in the secondary stretching was changed to 90°C, the temperature of the heat fixation was changed to 145°C, and the plasma treatment was performed only on one surface of the polyethylene microporous membrane.

A PET film to which an adhesive was attached was stuck on the surface of the substrate for a chromatography medium described above on the side where the plasma treatment was not performed to obtain the layered body.

[Example 9]

The substrate for a chromatography medium and the layered body were produced in the same manner as in Example 8 except that the SDBS coating was not performed to the polyethylene microporous membrane subjected to the plasma treatment.

[Comparative Example 1]

The substrate for a chromatography medium and the layered body were obtained in the same manner as in Example 1 except that the polyethylene composition prepared by mixing 7.5 parts by mass of UHMWPE and 17.5 parts by mass of HDPE was used, the stretching ratio in the MD direction in the primary stretching was changed to 1.1 times, in the secondary stretching, the stretching temperature in the MD direction was changed to 90°C, the stretching ratio in the MD direction was changed to 6.5 times, the stretching temperature in the TD direction was changed to 130°C, and the stretching ratio in the TD direction was changed to 13.5 times, and the temperature of the heat fixation was changed to 142°C.

[Comparative Example 2]

The polyethylene composition was prepared by mixing 10.2 parts by mass of UHMWPE and 6.8 parts by mass of HDPE. A mixed solvent was prepared by mixing 8 parts by mass of decalin and 75 parts by mass of liquid paraffin. The polyethylene solution was prepared by mixing the polyethylene composition and the mixed solvent so as to have a polymer concentration of 17% by mass. The polyethylene solution was extruded into a sheet shape from a die at a temperature of 153°C and then the extrudate was cooled in a water bath having a water temperature of 20°C to produce a gel-like sheet.

The gel-like sheet (base tape) described above was stretched in the MD direction with a stretch ratio of 3.0 times at a temperature of 90°C and then stretched in the TD direction with a stretch ratio of 9.0 times at a temperature of 105°C, immediately followed by the heat treatment (heat fixation) at 141°C. Next, the sheet was immersed in a two-tank methylene chloride bath successively for 30 seconds in each tank, thereby extracting the liquid paraffin. After the sheet was taken out from the methylene chloride bath, the methylene chloride was removed by drying in an atmosphere at a temperature of 40° C. The sheet was subjected to an annealing treatment while being conveyed on a roller heated to 120° C to obtain a biaxially stretched polyethylene microporous membrane.

The polyethylene microporous membrane described above was subjected to the plasma treatment and the SDBS coating as in Example 1 to obtain the substrate for a chromatography medium.

A PET film to which an adhesive was attached was stuck on one surface of the substrate for a chromatography medium described above to obtain a layered body.

[Comparative Example 3]

The polyethylene composition was prepared by mixing 3.4 parts by mass of UHMWPE and 13.6 parts by mass of HDPE. A mixed solvent was prepared by mixing 45 parts by mass of decalin and 38 parts by mass of liquid paraffin. The polyethylene solution was prepared by mixing the polyethylene composition and the mixed solvent so as to have a polymer concentration of 17% by mass. The polyethylene solution was extruded into a sheet shape from a die at a temperature of 157°C and then the extrudate was cooled in a water bath having a water temperature of 20°C to produce a gel-like sheet.

The gel-like sheet described above was subjected to pre-drying for 10 minutes in an atmosphere at a temperature of 95°C to obtain the base tape (a residual amount of the solvent in the base tape was kept less than 1%). Next, as the secondary stretching, the base tape was stretched in the MD direction with a stretch ratio of 5.5 times at a temperature of 90°C and then stretched in the TD direction with a stretch ratio of 10 times at a temperature of 105°C, immediately followed by the heat treatment (heat fixation) at 140°C. Subsequently, as in Comparative example 2, the sheet was immersed in the methylene chloride bath and subjected to the drying treatment and the annealing treatment to obtain the biaxially stretched polyethylene microporous membrane.

The polyethylene microporous membrane described above was subjected to the plasma treatment and the SDBS coating as in Example 1 to obtain the substrate for a chromatography medium.

A PET film to which an adhesive was attached was stuck on one surface of the substrate for a chromatography medium described above to obtain a layered body.

[Comparative Example 4]

The polyethylene microporous membrane before the hydrophilic treatment, produced in the Example 1, was used as the substrate for a chromatography medium.

A PET film to which an adhesive was attached was stuck on one surface of the substrate for a chromatography medium described above to obtain a layered body.

[Comparative Example 5]

A commercially available nitrocellulose membrane for immunochromatography (manufactured by Millipore Corp., SHF1200425) was used as the substrate for a chromatography medium. This commercially available product is a layered body in which a nitrocellulose membrane is layered on one side of a PET film.

Table 1 shows compositions and production conditions of each of the polyethylene microporous membranes of Examples 1 to 9 and Comparative examples 1 to 4.

[Table 1]

| Composition | | | Example 1 | Example 2 | Example 3 | Example 4 | Example 5 | Example 6 | Example 7 | Example 8 | Example 9 | Comparative example 1 | Comparative example 2 | Comparative example 3 | Comparative example 4 |
|---|---|---|---|---|---|---|---|---|---|---|---|---|---|---|---|
| | Decalin | parts by mass | 75 | 75 | 75 | 75 | 75 | 75 | 73 | 75 | 75 | 75 | 8 | 45 | 75 |
| | Paraffin | parts by mass | 0 | 0 | 0 | 0 | 0 | 0 | 0 | 0 | 0 | 0 | 75 | 38 | 0 |
| | UHMWPE | Mw | 4.6 million | 4.6 million | 4.6 million | 4.6 million | 4.6 million | 4.6 million | 4.6 million | 4.6 million | 4.6 million | 4.6 million | 4.6 million | 4.6 million | 4.6 million |
| | | parts by mass | 1.25 | 1.25 | 1.25 | 1.25 | 1.25 | 3.75 | 4.05 | 1.25 | 1.25 | 7.5 | 10.2 | 3.4 | 1.25 |
| | HDPE | Mw | 0.56 million | 0.56 million | 0.56 million | 0.56 million | 0.56 million | 0.56 million | 0.56 million | 0.56 million | 0.56 million | 0.56 million | 0.56 million | 0.56 million | 0.56 million |
| | | parts by mass | 23.75 | 23.75 | 23.75 | 23.75 | 23.75 | 21.25 | 22.95 | 23.75 | 23.75 | 17.5 | 6.8 | 13.6 | 23.75 |
| | Polymer concentration | % by mass | 25 | 25 | 25 | 25 | 25 | 25 | 27 | 25 | 25 | 25 | 17 | 17 | 25 |

(continued)

| | | | Example 1 | Example 2 | Example 3 | Example 4 | Example 5 | Example 6 | Example 7 | Example 8 | Example 9 | Comparative example 1 | Comparative example 2 | Comparative example 3 | Comparative example 4 |
|---|---|---|---|---|---|---|---|---|---|---|---|---|---|---|---|
| Extrusion | Die temperature | °C | 148 | 148 | 148 | 148 | 148 | 152 | 148 | 152 | 152 | 148 | 153 | 157 | 148 |
| | Cooling temperature | °C | 20 | 20 | 20 | 20 | 20 | 20 | 20 | 20 | 20 | 20 | 20 | 20 | 20 |
| | Pre-drying temperature | °C | 70 | 70 | 70 | 70 | 70 | 70 | 70 | 70 | 70 | 70 | - | 95 | 70 |
| | Pre-drying time | min. | 10 | 10 | 10 | 10 | 10 | 10 | 10 | 10 | 10 | 10 | - | 10 | 10 |
| | Primary stretching ratio | times | 1.4 | 1.4 | 1.8 | 1.2 | 1.2 | 1.2 | 1.4 | 1.4 | 1.4 | 1.1 | - | - | 1.4 |
| | Main drying temperature | °C | 57 | 57 | 57 | 57 | 57 | 57 | 57 | 57 | 57 | 57 | - | - | 57 |
| | Main drying time | min. | 5 | 5 | 5 | 5 | 5 | 5 | 5 | 5 | 5 | 5 | - | - | 5 |
| Stretching | MD stretching temperature | °C | 100 | 100 | 100 | 90 | 90 | 90 | 90 | 90 | 90 | 90 | 90 | 90 | 100 |
| | MD stretching ratio | times | 3.0 | 3.0 | 2.0 | 3.0 | 3.0 | 4.0 | 3.0 | 3.0 | 3.0 | 6.5 | 3.0 | 5.5 | 3.0 |
| | TD stretching temperature | °C | 125 | 125 | 120 | 125 | 125 | 125 | 125 | 125 | 125 | 130 | 105 | 105 | 125 |
| | TD stretching ratio | times | 9.0 | 9.0 | 4.4 | 9.0 | 9.0 | 9.0 | 9.0 | 9.0 | 9.0 | 13.5 | 9.0 | 10.0 | 9.0 |
| | Heat fixing temperature | °C | 127 | 127 | 127 | 145 | 145 | 144 | 144 | 145 | 145 | 142 | 141 | 140 | 127 |

(continued)

|  |  |  | Example 1 | Example 2 | Example 3 | Example 4 | Example 5 | Example 6 | Example 7 | Example 8 | Example 9 | Comparative example 1 | Comparative example 2 | Comparative example 3 | Comparative example 4 |
|---|---|---|---|---|---|---|---|---|---|---|---|---|---|---|---|
| Extraction | Extraction time | sec. | - | - | - | - | - | - | - | - | - | - | 60 | 60 | - |
|  | Drying temperature | °C | - | - | - | - | - | - | - | - | - | - | 40 | 40 | - |
|  | Annealing temperature | °C | - | - | - | - | - | - | - | - | - | - | 120 | 120 | - |
| Hydrophilic treatment | Plasma treatment |  | Both sides | Both sides | Both sides | Both sides | Both sides | Both sides | Both sides | One side | One side | Both sides | Both sides | Both sides | Not treated |
|  | Surfactant |  | SDBS | - | SDBS | SDBS | - | SDBS | SDBS | SDBS | - | SDBS | SDBS | SDBS | - |

<Production of Immunochromatographic Strip>

The substrates for chromatography media of Examples 1 to 9 and Comparative examples 1 to 5 were each used to produce the immunochromatographic strip having hCG (human chorionic gonadotropin) as a substance to be detected by the following procedures.

(1) Production of Chromatography Medium

The layered body of the substrate for a chromatography medium (hereinafter referred to as "substrate") and the PET film was cut out, along the MD direction and the TD direction of the substrate, into a rectangle having a length of 150 mm in the MD direction and a length of 25 mm in the TD direction. A phosphate buffer solution (pH 7.2) containing 0.5 mg/mL anti-hCG-a subunit antibodies (mouse monoclonal antibodies) was linearly applied (an application amount of 1 $\mu$L/cm) to the surface on the substrate side of the cut-out layered body in a direction parallel to the long side at a position 8 mm away from one of the long sides, followed by drying for 30 minutes in an atmosphere at a temperature of 50°C, to form the detection portion.

(2) Production of Labeling Substance Dispersion

To 10 mL of a dispersion prepared by diluting gold colloid (the labeling portion) having a particle diameter of 40 nm to a concentration of 60 $\mu$g/mL with a 50 mM $KH_2PO_4$ buffer solution (pH 7.0), 1 mL of anti-hCG antibodies (mouse monoclonal antibodies) (the binding portion) were added, and the mixture was allowed to stand at room temperature for 10 minutes. Next, 0.5 mL of an aqueous solution of 1% by mass polyethylene glycol (PEG, a weight-average molecular weight of 20,000) was added to the dispersion containing the gold colloid and the anti-hCG antibodies, followed by stirring. To this mixture, 1 mL of an aqueous solution of 10% by mass BSA (bovine serum albumin) was added and the mixture was further stirred. Next, the supernatant was removed by performing centrifugal separation at a centrifugal acceleration of 7,000 G for 15 minutes. Next, a 20mM Tris-hydrochloric acid buffer solution (Tris-HCl, pH 8.2) containing 0.05% by mass PEG (a weight-average molecular weight of 20,000), 0.009% by mass NaCl, 1% by mass BSA, and 0.095% by mass $NaN_3$ was added to the precipitate to disperse the labeling substances (the anti-hCG antibodies labeled with the gold colloid). The labeling substance dispersion was produced by the above procedures.

(3) Production of Conjugate Pad

To 0.7 mL of the labeling substance dispersion produced in the above, 2.1 mL of a Tris-hydrochloric acid buffer solution (Tris-HCl, pH 8.2) containing 0.05% by mass PEG (a weight-average molecular weight of 20,000) and 3.5% by mass sucrose was added, followed by stirring, to obtain a coating liquid. Next, the coating liquid was uniformly applied to a glass fiber-made pad (manufactured by Ahlstrom corp.) having a dimension of 150 mm x 8 mm x 400 $\mu$m, followed by drying with a vacuum dryer, to produce the conjugate pad.

(4) Production of Sample Pad

A Tris-hydrochloric acid buffer solution (Tris-HCl, pH 8.2) in an amount of 0.6 mL was uniformly applied to a cellulose-made pad (manufactured by Ahlstrom corp.) having a dimension of 150 mm x 18 mm x 340 $\mu$m, followed by drying at a temperature of 50°C for 1 hour, to produce the sample pad.

(5) Preparation of Absorption Pad

As the absorption pad, a filter paper (manufactured by Lohmann Technologies) having a size of 150 mm x 20 mm was prepared.

(6) Production of Immunochromatographic Strip

The chromatography medium, the conjugate pad, the sample pad, and the absorption pad, produced in the above, were placed one on another as shown in Fig. 1 and stuck to a backing sheet (manufactured by Lohmann Technologies, a size of 150 mm x 60 mm) to which an adhesive is applied on one side, thereby obtaining a complex sheet,. In this process, the overlapping width of the sample pad with the conjugate pad was set to 4 mm, the overlapping width of the conjugate pad with the chromatography medium was set to 2 mm, and the overlapping width of the chromatography medium with the absorption pad was set to 5 mm, and the detection portion of the chromatography medium was placed closer to the absorption pad than to the conjugate pad. The entire complex sheet was cut at a width of every 5 mm in the lengthwise direction to obtain strips each having a configuration shown in Fig. 1 (entire length in developing direction: 60 mm, width: 5 mm, the TD direction of the substrate corresponds to the developing direction of the sample).

<Performance Evaluation of Immunochromatographic Strip>

The following performance evaluation tests were performed in an atmosphere at a temperature of 24 °C and a relative humidity of 60%.

[Test Speed]

The hCG antigens (the substances to be detected) were diluted to 16.7 nkat with a phosphate buffer solution containing 1% by mass BSA and 0.095% by mass $NaN_3$ to produce a sample. The sample in an amount of 100 μL was dropped onto the sample pad of the immunochromatographic strip and developed, and the color developed in the detection portion was confirmed by visual observation. Starting from the moment when the sample was dropped onto the sample pad, the time (referred to as the detection time) is measured until the color development (red color) in the detection portion was confirmed by visual observation. The detection time was classified into the following three categories.

    A: detection time of less than 60 seconds
    B: detection time of from 60 seconds to less than 80 seconds
    C: detection time of 80 seconds or more

[Test Accuracy]

Besides the measurement of the detection time, the clarity of the color development (red color) in the detection portion was determined by visual observation, and the clarity was classified into the following three categories.

    A: red line can be clearly confirmed in detection portion
    B: red line can be confirmed in detection portion
    C: red line can be confirmed, but blurred in detection portion

Table 2 shows the physical properties of each of the polyethylene microporous membranes (PE microporous membranes) or the nitrocellulose membrane (NC membrane) of Examples 1 to 9 and Comparative examples 1 to 5, and the evaluation results of the immunochromatographic strips.

[Table 2]

| Substrate | | Plasma treatment | SDBS Yes/No | Membrane thickness (μm) | B as Weight (g/m²) | Porosity (%) | Gurley value (sec./100ml/μm) | BET specific surface area (m²/g) | Mean flow pore diameter (μm) | Contact angle of water (degrees) | Capillary flow time (sec./4cm) | Variance of capillary flow time | Test speed | Test accuracy |
|---|---|---|---|---|---|---|---|---|---|---|---|---|---|---|
| Example 1 | PE microporous membrane | Both sides | Yes | 95 | 8 | 91 | 0.003 | 7 | 3.0 | 0 | 77 | 2.5 | A | B |
| Example 2 | PE microporous membrane | Both sides | No | 95 | 8 | 91 | 0.003 | 7 | 3.0 | 28 | 164 | 5.1 | B | B |
| Example 3 | PE microporous membrane | Both sides | Yes | 425 | 44 | 89 | 0.002 | 5 | 2.4 | 0 | 71 | 2.3 | B | B |
| Example 4 | PE microporous membrane | Both sides | Yes | 78 | 14 | 81 | 0.01 | 3 | 1.4 | 9 | 96 | 1.1 | A | A |
| Example 5 | PE microporous membrane | Both sides | No | 78 | 14 | 81 | 0.01 | 3 | 1.4 | 19 | 101 | 6.4 | A | A |
| Example 6 | PE microporous membrane | Both sides | Yes | 110 | 12 | 89 | 0.02 | 8 | 0.8 | 9 | 187 | 3 | A | A |
| Example 7 | PE microporous membrane | Both sides | Yes | 93 | 17 | 81 | 0.1 | 12 | 0.2 | 13 | 231 | 5.3 | B | A |
| Example 8 | PE microporous membrane | One side | Yes | 29 | 4 | 84 | 0.02 | 4 | 2.5 | 2 | 168 | 1.3 | B | B |

| Substrate | | Plasma treatment | SDBS Yes/No | Membrane thickness | B as Weight | Porosity | Gurley value | BET specific surface area | Mean flow pore diameter | Contact angle of water | Capillary flow time | Variance of capillary flow time | Test speed | Test accuracy |
|---|---|---|---|---|---|---|---|---|---|---|---|---|---|---|
| | | | | $\mu$m | g/m$^2$ | % | sec./100ml/$\mu$m | m$^2$/g | $\mu$m | degrees | sec./4cm | | | |
| Example 9 | PE microporous membrane | One side | No | 29 | 4 | 84 | 0.02 | 4 | 2.5 | 48 | 164 | 6.3 | B | B |
| Comparative example 1 | PE microporous membrane | Both sides | Yes | 24 | 4 | 82 | 0.6 | 19 | 0.1 | 8 | > 600 | - | C | C |
| Comparative example 2 | PE microporous membrane | Both sides | Yes | 27 | 9 | 65 | 2.4 | 35 | 0.06 | 16 | > 600 | - | C | C |
| Comparative example 3 | PE microporous membrane | Both sides | Yes | 11 | 5 | 54 | 5.7 | 29 | 0.06 | 27 | > 600 | - | C | C |
| Comparative example 4 | PE microporous membrane | Not treated | No | 78 | 14 | 81 | 0.01 | 3 | 1.4 | 133 | Unmeasurable | - | C | C |
| Comparative example 5 | NC membrane | Not treated | Yes | 135 | - | - | - | - | - | 0 | 164 | 11.3 | A | A |

The entire disclosure of Japanese Patent Application No. 2017-180169 filed on September 20th, 2017 is incorporated herein by reference.

All publications, patent applications, and technical standards described in the present specification are incorporated herein by reference to the same extent as if each publication, patent application, or technical standard was specifically and individually indicated to be incorporated by reference.

REFERENCE SIGNS LIST

| | |
|---|---|
| A | Immunochromatographic strip |
| X | Developing direction |
| 1 | Chromatography medium |
| 2 | Sample pad |
| 3 | Conjugate pad |
| 4 | Absorption pad |
| 5 | Support plate |
| 11 | Detection portion |
| 101 | Substance to be detected |
| 102 | Labeling substance |
| 103 | Complex |
| 104 | Detection agent |

| | |
|---|---|
| Y | Direction to where water migrates |
| 6 | Cut-out piece of layered body of the substrate for a chromatography medium |
| 7 | Polypropylene plate |
| 8 | Adhesive tape |
| 9 | Beaker |
| 10 | Water |

**Claims**

1. A substrate for a chromatography medium that is included in an immunochromatographic strip, wherein:

    the substrate is formed of a hydrophilic polyolefin microporous membrane;
    the substrate has a contact angle one second after dropwise addition of water of from 0 degrees to 60 degrees on at least one surface of the substrate; and
    the substrate has a capillary flow time of from 5 seconds/4 cm to 300 seconds/4 cm.

2. The substrate for a chromatography medium according to claim 1, wherein the hydrophilic polyolefin microporous membrane has a membrane thickness of from 20 $\mu$m to 450 $\mu$m.

3. The substrate for a chromatography medium according to claim 1 or 2, wherein the hydrophilic polyolefin microporous membrane has a porosity of from 70% to 95%.

4. The substrate for a chromatography medium according to any one of claims 1 to 3, wherein the hydrophilic polyolefin microporous membrane has a BET specific surface area of from 1 $m^2$/g to 30 $m^2$/g.

5. The substrate for a chromatography medium according to any one of claims 1 to 4, wherein the hydrophilic polyolefin microporous membrane has a mean flow pore diameter of from 0.02 $\mu$m to 5 $\mu$m.

6. The substrate for a chromatography medium according to any one of claims 1 to 5, wherein the hydrophilic polyolefin microporous membrane is a microporous membrane in which a surfactant is attached to at least one of a surface of the polyolefin microporous membrane or an inner surface of a pore of the polyolefin microporous membrane.

7. The substrate for a chromatography medium according to any one of claims 1 to 6, wherein the hydrophilic polyolefin microporous membrane is a microporous membrane in which a plasma treatment is performed on at least one of a surface of the polyolefin microporous membrane or an inner surface of a pore of the polyolefin microporous membrane.

8. A chromatography medium comprising:

the substrate for a chromatography medium according to any one of claims 1 to 7; and
a detection portion which is arranged at the substrate for a chromatography medium and at which a detection agent that specifically binds to a substance to be detected is immobilized.

9. An immunochromatographic strip comprising the chromatography medium according to claim 8.

FIG.1

FIG.2

FIG.3

(a)

(b)

FIG.4

<div align="center">

**INTERNATIONAL SEARCH REPORT**

</div>

| | International application No. |
|---|---|
| | PCT/JP2018/031822 |

| A. CLASSIFICATION OF SUBJECT MATTER |
|---|
| Int.Cl. G01N33/543(2006.01)i, G01N33/545(2006.01)i |
| According to International Patent Classification (IPC) or to both national classification and IPC |

| B. FIELDS SEARCHED |
|---|
| Minimum documentation searched (classification system followed by classification symbols) |
| Int.Cl. G01N33/543, G01N33/545 |

| Documentation searched other than minimum documentation to the extent that such documents are included in the fields searched | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

| Electronic data base consulted during the international search (name of data base and, where practicable, search terms used) |
|---|
| |

| C. DOCUMENTS CONSIDERED TO BE RELEVANT | | |
|---|---|---|
| Category* | Citation of document, with indication, where appropriate, of the relevant passages | Relevant to claim No. |
| X/Y | JP 2009-503451 A (IDEXX LABORATORIES, INC.) 29 January 2009, entire text, all drawings, in particular, see claims, paragraphs [0029]-[0034], [0107], etc. & US 2007/0020699 A1, entire text, all drawings, in particular, see claims, paragraphs [0038]-[0044], [0172], etc. & US 2007/0020700 A1 & WO 2007/011936 A2 & EP 1907850 A2 | 1-5,7-9/6 |

☒ Further documents are listed in the continuation of Box C.  ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 20 November 2018 (20.11.2018) | 04 December 2018 (04.12.2018) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
|---|
| PCT/JP2018/031822 |

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2002-510048 A (EPITOPE, INC.) 02 April 2002, entire text, all drawings, in particular, see page 12, lines 6-25, page 20, line 4 to page 21, line 15, fig. 1, 2, etc. & US 2002/0155029 A1, entire text, all drawings, in particular, see claims, paragraphs [0013], [0051]-[0054], fig. 1, 2, etc. & US 2002/0192839 A1 & US 2007/0116597 A1 & US 2010/0239458 A1 & US 2012/0149124 A1 & US 2009/0170072 A1 & WO 1999/050656 A1 & EP 1086372 A1 & EP 1696236 A2 | 6 |
| A | WO 2014/077142 A (KONICA MINOLTA, INC.) 22 May 2014, entire text, all drawings, in particular, see paragraph [0080], etc. (Family: none) | 1-9 |
| A | JP 2007-506115 A (QUIDEL CORPORATION) 15 March 2007, see entire text, all drawings & US 2005/0130120 A1 & US 2009/0029453 A1 & US 2010/0159444 A1 & US 2014/0199685 A1 & US 2016/0047005 A1 & US 2018/0030552 A1 & WO 2005/031355 A1 & EP 1664781 A1 | 1-9 |
| A | JP 2017-067542 A (BESERU KK) 06 April 2017, see entire text, all drawings (Family: none) | 1-9 |

Form PCT/ISA/210 (continuation of second sheet) (January 2015)

**REFERENCES CITED IN THE DESCRIPTION**

**Patent documents cited in the description**

- JP 2008292326 A
- JP 2009150869 A
- JP 2010261912 A

- JP 2013174607 A
- JP 2014062820 A
- JP 2017180169 A